# EUROPEAN PATENT SPECIFICATION

(11) **EP 3 067 040 B1**
(45) Date of publication and mention of the grant of the patent: **27.09.2017**
(21) Application number: 16159963.4
(22) Date of filing: 11.03.2016
(51) Int. Cl.: A61K 8/60, A61Q 19/08

(54) **MIXTURE OF ACTIVE INGREDIENTS FOR COMPOSITIONS FOR COSMETIC USE**
MISCHUNG VON WIRKSTOFFEN FÜR ZUSAMMENSETZUNGEN ZUR KOSMETISCHEN VERWENDUNG
MÉLANGE DE PRINCIPES ACTIFS POUR DES COMPOSITIONS À USAGE COSMÉTIQUE

(30) Priority: 13.03.2015 IT PD20150061
(43) Date of publication of application: 14.09.2016
(73) Proprietor: Kialab S.r.l., 36100 Vicenza (VI) (IT)
(72) Inventor: ARMADA, Massimo, 20017 Rho (MI) (IT); ALBERTI, Federica, 29027 Podenzano (PC) (IT)
(74) Representative: Gallo, Luca

(56) References cited:
- EP-A1- 2 204 162
- WO-A1-02/20622
- WO-A1-95/05155
- JP-A- H01 163 110

## Description

### Field of application

The present invention regards a mixture of active ingredients for compositions for cosmetic use, according to the independent claim 1.

The mixture of active ingredients, object of the present invention, is intended to be advantageously employed in cosmetic formulations, preferably aimed to oppose the aging processes, in particular skin aging.

The invention is therefore inserted in the field of the cosmetic industry and more generally in the field of production of products for treating people.

### State of the art

In the cosmetics field, constant attention is aimed for identifying and implementing new active ingredients adapted to oppose the aging processes, in particular skin aging.

Such aging processes are directly correlated both with the hydration capacity of the skin, hence with the bioavailability of water in cells, and with the synthesis by the cells of substances indispensable for maintaining skin turgor, such as in particular Elastin, Collagen and Fibrillin.

In addition, in the scope of production of cosmetic formulations, particular attention is directed towards the safety of the components, whether these are excipients or active principles, which must allow limiting adverse skin reactions as much as possible.

In particular, for some time there has been the need to reduce the use of components of chemical derivation in favor of components of natural origin or biotechnology derivatives, i.e. not obtained via chemical synthesis. Such need is derived both from market reasons, given that such substances of natural origin are more requested by consumers, and from the desire to use, in cosmetic formulations, molecules identical to those present in the human organism or in breast milk, in particular in order to use such formulations on sensitive skin and in baby care.

It has also for some time been verified that the natural excipients and active principles are capable of establishing an interaction with the tissues on which they are applied, both at the biochemical and metabolic level, that is already recognized and hence more effective than active principles and excipients derived from chemical synthesis and not identically present in nature. For such purpose, in recent years, components of carbohydrate origin have been used in various cosmetic formulations; such components are tolerated by the skin, and are used both as excipients, in particular emulsifiers and surfactants, and as active principles, in particular as hydrating agents. More in detail, such components of carbohydrate origin are admixed with cosmetic compositions usually for their hydration capacity, i.e. for their capacity to attract and bond water, necessary for implementing cellular metabolic reactions.

For example, known from the patent EP 2204162 is a cosmetic composition for topical use comprising one or more monosaccharides, selected from between mannose and rhamnose, adapted to oppose the signs of skin aging and in particular epidermal and dermal atrophy tied to aging. According to the teaching reported in the patent EP 2204162, such monosaccharides are capable of stimulating the synthesis of Collagen and, in particular, the synthesis of Procollagen type 1.

Such cosmetic composition has nevertheless in practice proven to be not free of drawbacks.

The main drawback lies in the poor capacity of such composition to ensure a high anti-aging effectiveness over time. The monosaccharides contained in the composition described briefly above are in fact able to ensure an optimal level of hydration, but are unable to sufficiently stimulate the synthesis of some of the substances indispensable for maintaining skin elasticity and hydration, such as in particular Elastin.

The patent JP H01163110 describes a cosmetic composition of known type in order to prevent skin aging, which comprises components selected from among Sialic Acid and derivatives of the latter such as N-acetylneuraminic Acid. WO 02/20622 discloses mixtures of fucose-based oligosaccharides for use against skin aging. Nevertheless, also the latter solution of known type is not able to oppose the skin aging processes in a particularly efficient manner.

### Presentation of the invention

In this situation, the problem underlying the present invention is that of providing a mixture of active ingredients for compositions for cosmetic use which is capable of effectively opposing the skin aging processes.

Another object of the present invention is to provide a mixture of active ingredients for compositions for cosmetic use which is capable of performing an anti-aging action that endures over time.

A further object of the present invention is to provide a mixture of active ingredients for compositions for cosmetic use which is capable of facilitating an optimal hydration and simultaneously stimulating the synthesis of substances at the skin that are adapted to maintain skin turgor.

Another object of the present invention is to provide a mixture of active ingredients for compositions for cosmetic use which is well-tolerated and minimally allergenic.

These and still other objects are all achieved by the mixture of active ingredients for compositions for cosmetic use, according to the enclosed claims.

### Brief description of the drawings

The technical characteristics of the invention, according to the aforesaid objects, can be clearly seen in the contents of the below-reported claims and the advantages thereof will be more evident in the following detailed description, made with reference to the enclosed graphs, which refer to a merely exemplifying and non-limiting embodiment, in which:
- Figure 1 shows a first graph reporting the progression of the radial distribution function between the oxygen elements of water with respect to the distance for various aqueous solutions of monosaccharides or of oligosaccharides and for pure water;
- figure 2 shows an enlargement of the first peak of the graph of Figure 1;
- Figure 3 shows a second graph reporting the progression of the overall solvent-accessible surface area (SASA) of the molecules of Fucose, Arabinose, Galactose, 6'-Sialyllactose and 2'-Fucosyllactose in aqueous solution;
- Figures 4a and 4b respectively illustrate a 3D simulation of an aqueous solution of oligosaccharide (2'-Fucosyllactose) and a 3D simulation of an aqueous solution of a mixture of monosaccharides (Arabinose and Fucose) and oligosaccharide (2'-Fucosyllactose) in 3:1 ratio;
- Figures 5a and 5b show the diagrams relative to the gene expression of Decorin and Collagen IV respectively after 24 hours and after 48 hours of treatment for two different mixtures of active ingredients for compositions for cosmetic use, and more in detail are referred to mixtures of only monosaccharides and to mixtures of monosaccharides and oligosaccharides in 3:1 ratio;
- Figures 6a and 6b show the diagrams relative to the gene expression of Fibrillin and Elastin respectively after 24 hours and after 48 hours of treatment for two different mixtures of active ingredients for compositions for cosmetic use, and more in detail are referred to mixtures of only monosaccharides and to mixtures of monosaccharides and oligosaccharides in 3:1 ratio.

### Detailed description

The mixture of active ingredients for compositions for cosmetic use, object of the present invention, is intended to be employed in cosmetic and/or pharmaceutical context for the production of products for personal treatment and, more in detail, for the production of products for topical use adapted to oppose aging processes.

The mixture of active ingredients for compositions for cosmetic use according to the present invention was in particular designed for opposing skin aging processes; nevertheless, it can also find use in cosmetic and/or pharmacological formulations intended for example for the treatment of hair, nails and mucous membranes, without departing from the protective scope defined by the present patent.

With the expression "composition for cosmetic use", it is intended to indicate herein any substance or preparation intended to be placed in contact with various parts of the human body, such as in particular the epidermis, the nails, hair and the lips, with the main purpose of protecting and/or preserving them in good conditions, according to the definition provided by the European Directive No. 1223/2009.

Even if the mixture of active ingredients according to the present invention was implemented mainly for cosmetic use, it can also be admixed with pharmaceutical and medicinal active principles or be inserted in medicinal formulations for the purpose of providing the latter with the beneficial properties described hereinbelow, without departing from the protective scope defined by the present patent.

In particular the mixture of active ingredients in accordance with the invention can be used for obtaining compositions such as creams, emulsions, lotions, cream deodorants, make-up products and more generally any product for topical use and preferably for dermatological use.

Hereinbelow in the present description, in order to facilitate the exposition reference will be made to a mixture of active ingredients for compositions for cosmetic use intended for skin application; nevertheless, it is intended that such compositions can also be intended for other applications, as previously specified, without departing from the protective scope defined by the present patent.

As is better explained hereinbelow, the mixture of active ingredients in accordance with the present invention is intended to be employed for making compositions adapted to facilitate the hydration of the tissues on which they are applied and also capable of effectively facilitating the synthesis, by such tissues, of substances indispensable for maintaining the elasticity and tonicity of the tissues themselves which, following natural aging processes, tend to be synthesized in lesser quantities.

The mixture of active ingredients for compositions for cosmetic use (and in particular advantageously for compositions for topical use as previously specified) according to the present invention comprises two or more carbohydrate components, including at least one monosaccharide and at least one oligosaccharide characterized in that the weight ratio between said at least one monosaccharide and said at least one oligosaccharide present in said mixture is comprised between 70:30 and 90:10 ; wherein said at least one monosaccharide comprises Arabinose and Fucose and said at least one oligosaccharide comprises 2-Fucosyllactose ; wherein, in addition, said at least two carbohydrate components comprise a weight percentage of said Arabinose comprised between about 40% and 60% , a weight percentage of said Fucose comprised between about 15% and 35% and a weight percentage of said 2-Fucosyllactose comprised between about 15% and 35%. More in detail, the mixture of active ingredients according to the invention preferably comprises, as carbohydrate components, only monosaccharides and oligosaccharides.

According to the present invention, the weight ratio between the monosaccharide (or the monosaccharides) and the oligosaccharide (or the oligosaccharides), present in the mixture of active ingredients, is comprised between 70:30 and 90:10.

More clearly, a weight percentage comprised between 70% and 90% of the carbohydrate components comprised in the mixture of active ingredients is constituted by one or more monosaccharides and a weight percentage of the carbohydrate components comprised between 10% and 30% is constituted by one or more oligosaccharides.

Indeed, it was surprisingly verified that the combination of monosaccharides and oligosaccharides in the above-indicated proportions allows considerably improving the hydration capacity of the skin, facilitating the bioavailability of water at the cellular level, and also allows facilitating the synthesis by the cells of the tegumentary apparatus of substances indispensable for maintaining the elasticity, tone and appearance of the skin, such as in particular Elastin, Collagen, Decorin and Fibrillin, generically indicated hereinbelow with the expression "anti-aging substances" for the sake of exposition simplicity.

In particular, mixtures of active ingredients for compositions for cosmetic use comprising one or more monosaccharides and one or more oligosaccharides, in a weight ratio with respect to each other of about 3:1, have proven particularly effective in facilitating skin hydration and in opposing skin aging processes.

The combination of monosaccharides and oligosaccharides in different proportions with respect to those indicated above does not allow obtaining the same performance characteristics of the mixture of ingredients for compositions for cosmetic use according to the invention.

Indeed it has been detected that although greater concentrations of oligosaccharides with respect to those indicated above more greatly facilitate the retention of the water molecules within the cells, and hence the hydration, they are unable to stimulate the production of anti-aging substances in an equally effective manner.

Both the oligosaccharides and the monosaccharides are osmoprotectants and are therefore able to confer a level of order to the water and reduce the self-diffusion coefficient of the latter.

Indeed, osmoprotectants belong to the category of cosolvents termed "structure forming", in reference to their capacity to stabilize the microscopic structure of water, i.e. in particular the network of hydrogen bonds that are formed between the water molecules. Osmoprotectants also induce the decrease of the self-diffusion coefficient of water, thus affecting not only the structural properties but also the dynamic properties thereof.

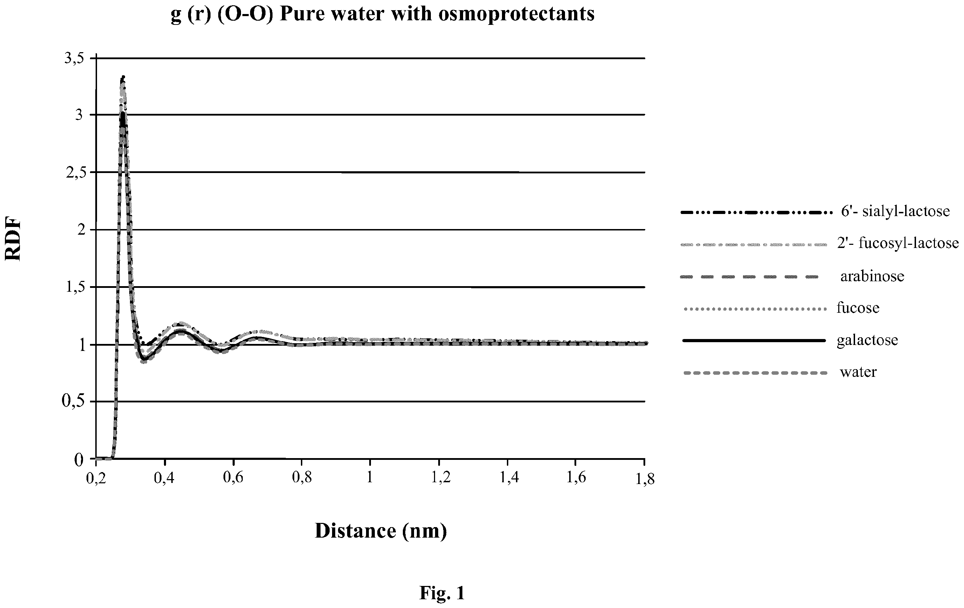

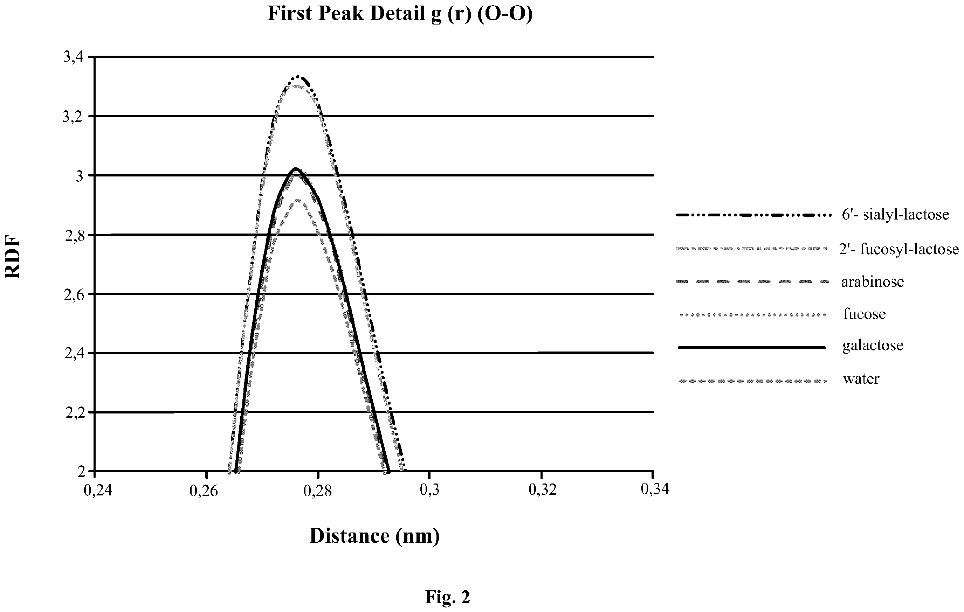

The size of the osmoprotection effects is greater for the oligosaccharides and much more limited for the monosaccharides. Such greater osmoprotection effectiveness of the oligosaccharides with respect to the monosaccharides was verified by means of tests in silico, in which simulations were carried out of molecular dynamics of aqueous solutions containing a monosaccharide (in particular Fucose, Arabinose or Galactose) or an oligosaccharide (in particular 6'-Sialyllactose or 2'-Fucosyllactose), each of which in 0.5 M concentration. The obtained results were compared with those relative to pure water. In particular, the radial distribution function was calculated between the oxygen elements of water g(r) (o-o) whose progression provides information on the macroscopic structure of water and on its order level, allowing the evaluation of how the presence of different osmoprotectants affects the network of hydrogen bonds responsible for the microscopic structure of the solvent. The enclosed Figures 1 and 2 respectively report the progression of the radial distribution function between the oxygen elements of water with respect to the distance for each of the considered solutions and for the water, and an enlargement of the first peak visible in Figure 1, relative to the first solvation shell of water, i.e. to the hydrogen bonds that a molecule forms with the molecules immediately adjacent thereto. As is clearly visible in the aforesaid figures, the oligosaccharides (6'-Sialyllactose and/or 2'-Fucosyllactose) have a clearly greater effect with respect to the monosaccharides (Fucose, Arabinose and Galactose).

The effectiveness of osmoprotection of the oligosaccharides is therefore greater than the effectiveness of osmoprotection of the monosaccharides.

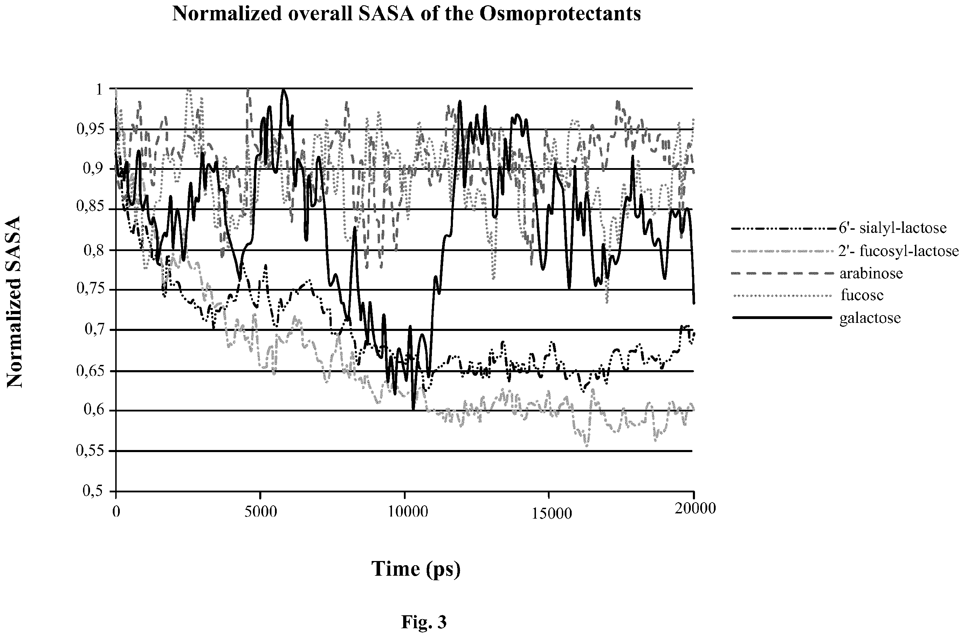

Oligosaccharides nevertheless have a tendency to aggregate and form clusters of molecules in solution that is significantly higher than that of the monosaccharides, as can be seen in the graph reported in Figure 3, which reports the progression of the overall solvent-accessible surface area (SASA) of the molecules of Fucose, Arabinose, Galactose, 6'-Sialyllactose and 2'-Fucosyllactose in aqueous solution, which is significantly lower for the oligosaccharides with respect to the monosaccharides.

The formation of such aggregates - even if it facilitates the retention of water, which is substantially trapped in the aggregates themselves, and hence the hydration - limits the capacity of the oligosaccharides to carry out their protection function with regard to membranes and proteins. In addition, the water thus trapped has poor bioavailability, and hence is substantially unavailable to the organism for cellular metabolic reactions.

The presence of oligosaccharides in the composition according to the invention in a weight percentage greater than the maximum weight percentage reported above therefore is unable to facilitate the production, in an equally effective manner of anti-aging substances such as Elastin, Decorin, Fibrillin and Collagen, which are essential for opposing the signs of skin aging. Indeed, for the synthesis of such substances, it is necessary to have cellular wellbeing, which is strictly tied to the availability of water, since substantially all metabolic processes require water and nutriments.

Conversely, the presence of oligosaccharides in the composition according to the invention in a weight percentage lower than the above-reported minimum weight percentage is unable to ensure a suitable hydration, since the water molecules are overly mobile, not being sufficiently retained the cellular environment by the carbohydrate compounds.

The mixture of one or more monosaccharides and one or more oligosaccharides in a weight ratio, comprised in the interval identified in accordance with the present invention, allows simultaneously ensuring an optimal hydration level and an optimal stimulation level of the metabolic processes that lead to the formation of the substances set to oppose skin aging processes.

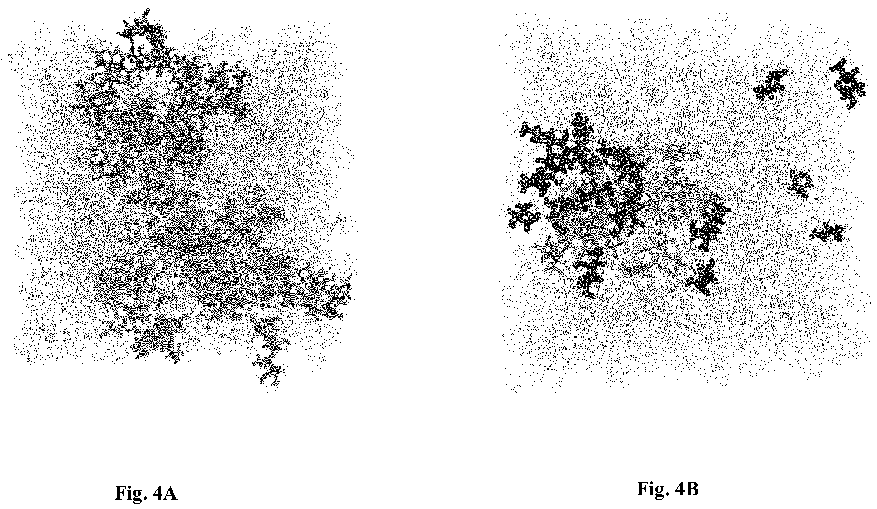

This is due, in particular, to the synergistic action of the monosaccharide with the oligosaccharide in the proportions according to the invention, due to the fact that the monosaccharide, inserted between the oligosaccharide molecules, limits the aggregation and increases the bioavailability thereof, as is clear from the example illustrated in figures 4a and 4b. In particular, figure 4a illustrates a simulation in 3D of an aqueous solution of oligosaccharide (in particular 2'-Fucosyllactose), where the formation of a large aggregate of molecules is evident. Figure 4b illustrates a simulation in 3D of an aqueous solution of the mixture of monosaccharides (in particular Fucose and Arabinose) and oligosaccharide (in particular 2'-Fucosyllactose) in 3:1 proportion, where the molecules of monosaccharides (depicted with dashed edge) - in addition to not giving rise to aggregates - are inserted between the oligosaccharide molecules, limiting the aggregation and increasing the bioavailability thereof.

The aforesaid performance characteristics of the mixture of active ingredients according to the present invention are achieved due to the optimal management of water, i.e. in particular due to the equilibrium that is established between the retention of water, desired for facilitating hydration, and release thereof, desired for ensuring the bioavailability thereof for the cellular metabolic processes. Such equilibrium is achieved by the mixture of active ingredients according to the invention due to the optimal proportions identified between monosaccharides and oligosaccharides.

Advantageously, the monosaccharide (or the monosaccharides) and the oligosaccharide (or the oligosaccharides) present in the mixture according to the present invention have a purity greater than 95% and preferably greater than 96%-98%.

Indeed, as specified above, the effectiveness of the mixture of active ingredients according to the invention is due to the functional equilibrium that is established between the monosaccharides and the oligosaccharides. The interaction capacities of the carbohydrate components, i.e. of the monosaccharides and the oligosaccharides, present in the composition with the water molecules and with the proteins that intervene in the cellular metabolic reactions, is strongly tied to the molecular structure of the carbohydrate components themselves.

The chemical structure of the carbohydrate molecules, i.e. for example the number of hydroxyl groups -OH present in the molecule or the steric arrangement thereof, is in fact essential for determining the reactivity with the medium.

Therefore, a high level of purity of such components ensures the obtainment of the desired functional characteristics.

In addition, the use of carbohydrate components with high purity ensures a greater specificity of action of such components, thus preventing the presence of other molecules or parts thereof in the mixture according to the invention which do not actively contribute to the hydration and anti-aging effectiveness. Finally, the monosaccharides and the oligosaccharides with a high purity level, having lower molecular weight, have a greater penetration capacity, i.e. a greater capacity to reach the site of activity.

In accordance with a particularly advantageous embodiment of the composition, object of the invention, the monosaccharides and the oligosaccharides are obtained in a biotechnological way. This in fact allows obtaining the molecules of the desired carbohydrate components with a high level of purity.

The derivation of the molecules of the carbohydrate components, for example starting from raw plant materials, would involve the presence of overly high percentages of impurities, and would therefore not allow the obtainment of a purity level sufficient for ensuring the above-described performance characteristics. The monosaccharide present in the mixture of active ingredients according to the invention comprises Arabinose and Fucose. The oligosaccharide present in the composition according to the invention comprises 2'-Fucosyllactose. Preferably, the mixture of active ingredients can comprise multiple oligosaccharides, i.e. in particular a combination of two or more oligosaccharides selected from among those indicated above.

The mixture of active ingredients for compositions for cosmetic use according to the invention comprises two monosaccharides, Arabinose and Fucose, and one oligosaccharide, 2'-Fucosyllactose.

According to the invention, the carbohydrate components comprise a weight percentage of Arabinose comprised between about 40% and 60%, a weight percentage of Fucose comprised between about 15% and 35% and a weight percentage of 2'-Fucosyllactose comprised between about 15% and 35%. The mixture in accordance with the invention has allowed obtaining surprising results regarding both the capacity of hydration and the capacity of stimulation of the synthesis of the anti-aging substances even over the long term, thus ensuring the performance of an anti-aging action even days after the application of the composition, as demonstrated in the example reported hereinbelow.

Preferably, in accordance with a particularly advantageous embodiment of the present invention, the carbohydrate components comprise a weight percentage of Arabinose of about 50%, a weight percentage of Fucose of about 25% and a weight percentage of 2'-Fucosyllactose of about 25%.

Also forming the object of the present invention is a composition for cosmetic use comprising the aforesaid mixture of active ingredients.

The carbohydrate components (monosaccharides and oligosaccharides) of the mixture of active ingredients are contained in the composition for cosmetic use according to the invention in a weight percentage equal to at least 0.01%. Preferably, the weight percentage of such carbohydrate components in the composition for cosmetic use is comprised between 0.01 % and 10%.

### Example

Hereinbelow, by way of a non-limiting example, we report the results of tests that were conducted by directly applying the mixture of active ingredients according to the present invention on the surface of a reconstructed epidermal tissue in a physiological situation, in order to highlight the biological effects and dermal and epidermal action mechanism thereof, in a situation that mimics and is predictive of the in vivo treatment mode. More in detail, the tests were conducted on a model of multi-layered human skin formed by keratinocytes and fibroblasts and comprising epidermis, basal membrane and dermis.

The object of the tests was to measure the capacity of different mixtures for compositions for cosmetic use to stimulate, at the genomic level, the synthesis of specific markers of the dermal-epidermal structure. In particular, the variation over time was evaluated of the concentration of Collagen IV (COL IV), of Decorin (DCN), of Elastin (ELN) and of Fibrillin 1 (FBN1).

The present example and the enclosed figures report the results obtained following the application of two different mixtures of monosaccharides and oligosaccharides, including a first in accordance with the present invention, indicated in the figures with the identification (MTAFFL722) and comprising as carbohydrate components two monosaccharides and one oligosaccharide in a weight ratio with respect to each other of 3:1 (40-60% Arabinose, 15-35% Fucose and 15-35% 2'-Fucosyllactose). The second mixture (not in accordance with the present invention), indicated in the figures with the identification (MBAF72), comprises as carbohydrate components only monosaccharides (65-85% Arabinose and 15-35% Fucose). Finally, a control composition was also evaluated, indicated in the figures with the identification (CN), constituted by a saline solution of 0.9% NaCl.

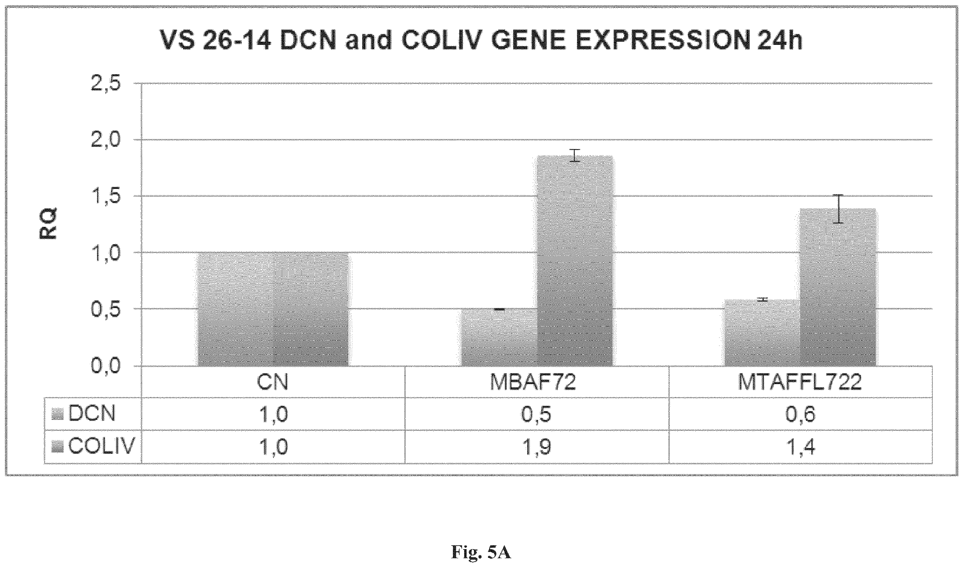

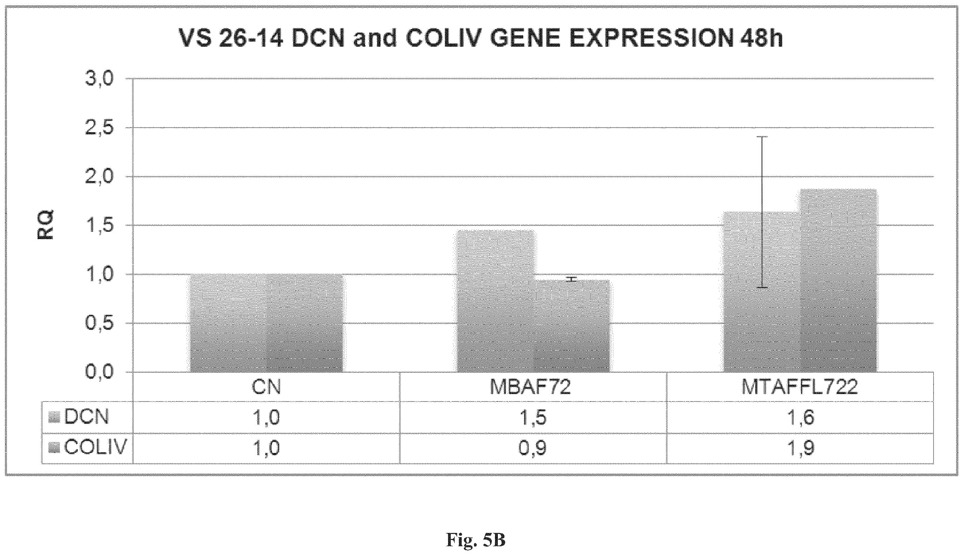

Figures 5a and 5b report the diagrams relative to the gene expression of Decorin and Collagen IV respectively after 24 hours and after 48 hours of treatment for the different compositions considered in this example.

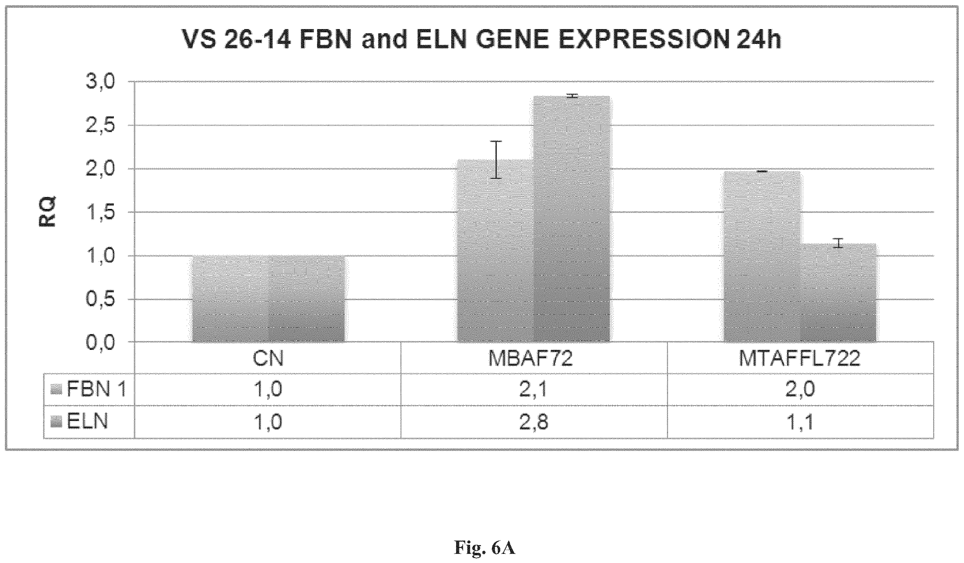

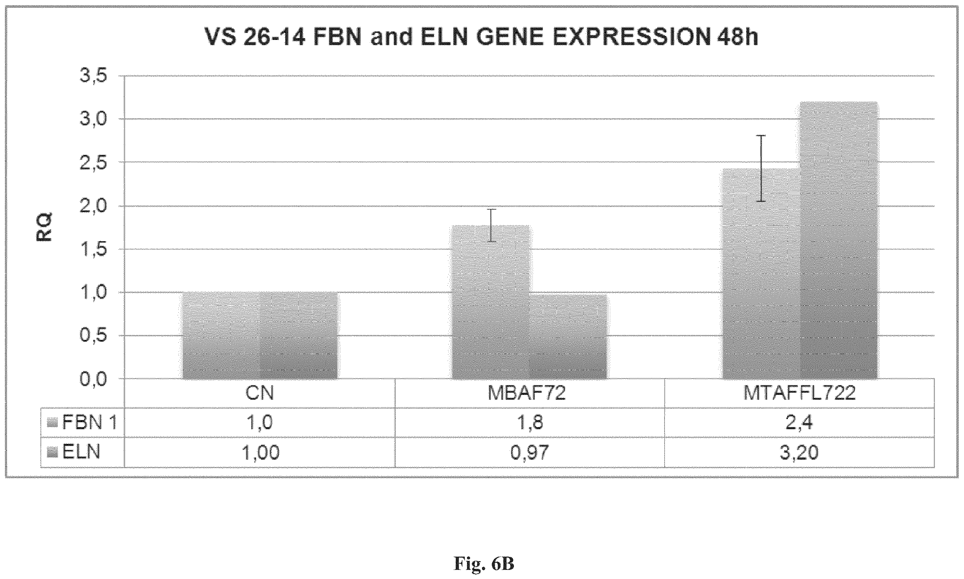

Figures 6a and 6b report the diagrams relative to the gene expression of Fibrillin and Elastin respectively after 24 hours and after 48 hours of treatment for the different mixtures considered in this example.

The diagrams reported in Figures 5a-6b clearly show that the mixture in accordance with the present invention is capable of effectively stimulating the production of anti-aging substances not only over a short-term period but also over a long-term period, i.e. at 48 hours, which is a particularly important aspect.

The mixture containing only monosaccharides, while demonstrating more promising gene expression at 24 hours, is unable to perform an anti-aging action in the long term, i.e. at 48 hours.

It can be surprisingly observed that the mixture of active ingredients, according to the present invention, is able to stimulate in a particularly effective manner the synthesis of Elastin, responsible for the tensile force of the skin, and of Fibrillin, essential for stabilizing the structure of the elastic fibers, which at 48 hours reach values higher than the values reached by the same at 24 hours following the application of the mixture containing only monosaccharides.

The finding thus conceived therefore attains the pre-established objects.

## Claims

1. Mixture of active ingredients for compositions for cosmetic use, such mixture comprising at least two carbohydrate components, at least one of which being a monosaccharide and at least one of which being an oligosaccharide, **characterized in that** the weight ratio between said at least one monosaccharide and said at least one oligosaccharide present in said mixture is comprised between 70:30 and 90:10;
wherein said at least one monosaccharide comprises Arabinose and Fucose and said at least one oligosaccharide comprises 2'-Fucosyllactose;
wherein, in addition, said at least two carbohydrate components comprise a weight percentage of said Arabinose comprised between about 40% and 60%, a weight percentage of said Fucose comprised between about 15% and 35% and a weight percentage of said 2'-Fucosyllactose comprised between about 15% and 35%.

2. Mixture of active ingredients according to claim 1, **characterized in that** said at least one monosaccharide and said at least one oligosaccharide are in a weight ratio with respect to each other of about 3:1.

3. Mixture of active ingredients according to claim 2, **characterized in that** said at least two carbohydrate components comprise a weight percentage of about 50% of said Arabinose, a weight percentage of about 25% of said Fucose and a weight percentage of about 25% of said 2'-Fucosyllactose.

4. Mixture of active ingredients according to any one of the preceding claims, **characterized in that** said at least one monosaccharide and said at least one oligosaccharide have a purity greater than 95%.

5. Mixture of active ingredients according to claim 4, **characterized in that** said at least one monosaccharide and said at least one oligosaccharide have a purity greater than 96-98%.

6. Mixture of active ingredients according to any one of the preceding claims, **characterized in that** said at least one monosaccharide and said at least one oligosaccharide are obtained in a biotechnological way.

7. Composition for cosmetic use, **characterized in that** it comprises a mixture of active ingredients according to any one of the preceding claims, said at least two carbohydrate components of said mixture of active ingredients being contained in said composition in a weight percentage equal to at least 0.01%.

8. Composition for cosmetic use according to claim 7, **characterized in that** said at least two carbohydrate components are contained in said composition in a weight percentage comprised between 0.01 % and 10%.

## Patentansprüche

1. Mischung von Wirkstoffen für Zusammensetzungen zur kosmetischen Verwendung, die mindestens zwei Kohlenhydratbestandteile umfasst, davon mindestens ein Monosaccharid und mindestens ein Oligosaccharid, **dadurch gekennzeichnet, dass** das Gewichtsverhältnis zwischen dem genannten mindestens einen Monosaccharid und dem genannten mindestens einen Oligosaccharid in der genannten Mischung zwischen 70:30 und 90:10 beträgt;
bei der das genannte mindestens eine Monosaccharid Arabinose und Fucose und das genannte mindestens eine Oligosaccharid 2'-Fucosyllactose umfasst;
bei der außerdem die genannten mindestens zwei Kohlenhydratbestandteile einen Gewichtsanteil zwischen ca. 40 % und 60 % der genannten Arabinose, einen Gewichtsanteil zwischen ca. 15 % und 35 % der genannten Fucose und einen Gewichtsanteil zwischen ca. 15 % und 35 % der genannten 2'-Fucosyllactose umfassen.

2. Mischung von Wirkstoffen nach Anspruch 1, **dadurch gekennzeichnet, dass** das genannte mindestens eine Monosaccharid und das genannte mindestens eine Oligosaccharid in einem Gewichtsverhältnis von ca. 3:1 vorliegen.

3. Mischung von Wirkstoffen nach Anspruch 2, **dadurch gekennzeichnet, dass** die genannten mindestens zwei Kohlenhydratbestandteile einen Gewichtsanteil von ca. 50 % der genannten Arabinose, einen Gewichtsanteil von ca. 25 % der genannten Fucose und einen Gewichtsanteil von ca. 25 % der genannten 2'-Fucosyllactose umfassen.

4. Mischung von Wirkstoffen nach einem beliebigen der vorangegangenen Ansprüche, **dadurch gekennzeichnet, dass** das genannte mindestens eine Monosaccharid und das genannte mindestens eine Oligosaccharid eine Reinheit von über 95 %. aufweisen.

5. Mischung von Wirkstoffen nach Anspruch 4, **dadurch gekennzeichnet, dass** das genannte mindestens eine Monosaccharid und das genannte mindestens eine Oligosaccharid eine Reinheit von über 96-98%. aufweisen.

6. Mischung von Wirkstoffen nach einem beliebigen der vorangegangenen Ansprüche, **dadurch gekennzeichnet, dass** das genannte mindestens eine Monosaccharid und das genannte mindestens eine Oligosaccharid mit biotechnologischen Methoden erzielt werden.

7. Zusammensetzung zur kosmetischen Verwendung, **dadurch gekennzeichnet, dass** sie eine Mischung von Wirkstoffen nach einem beliebigen der vorangegangenen Ansprüche umfasst, wobei die genannten mindestens zwei Kohlenhydratbestandteile der genannten Mischung von Wirkstoffen in der genannten Zusammensetzung in einem Gewichtsanteil in Höhe von mindestens 0,01 % enthalten sind.

8. Zusammensetzung zur kosmetischen Verwendung nach Anspruch 7, **dadurch gekennzeichnet, dass** die genannten mindestens zwei Kohlenhydratbestandteile in der genannten Zusammensetzung in einem Gewichtsanteil zwischen 0,01 % und 10 % enthalten sind.

## Revendications

1. Mélange de principes actifs pour des compositions à usage cosmétique, ledit mélange comprenant au moins deux composants glucidiques, dont au moins un monosaccharide et au moins un oligosaccharide, **caractérisé en ce que** le rapport en poids entre ledit au moins un monosaccharide et ledit au moins un oligosaccharide présents dans ledit mélange est compris entre 70:30 et 90:10 ;
où ledit au moins un monosaccharide comprend de l'arabinose et du fucose et ledit au moins un oligosaccharide comprend 2'-fucosyllactose ;
où, en outre, lesdits au moins deux composants glucidiques comprennent un pourcentage en poids compris entre environ 40% et 60% dudit arabinose, un pourcentage en poids compris entre environ 15% et 35% dudit fucose et un pourcentage en poids compris entre environ 15% et 35% dudit 2'- fucosyllactose.

2. Mélange de principes actifs selon la revendication 1, **caractérisé en ce que** ledit au moins un monosaccharide et ledit au moins un oligosaccharide ont un rapport en poids d'environ 3:1 l'un par rapport à l'autre.

3. Mélange de principes actifs selon la revendication 2, **caractérisé en ce que** lesdits au moins deux composants glucidiques comprennent un pourcentage en poids d'environ 50% dudit arabinose, un pourcentage en poids d'environ 25% dudit fucose et un pourcentage en poids d'environ 25% dudit 2'- fucosyllactose.

4. Mélange de principes actifs selon l'une des revendications précédentes, **caractérisé en ce que** ledit au moins un monosaccharide et ledit au moins un oligosaccharide ont une pureté supérieure à 95%.

5. Mélange de principes actifs selon la revendication 4, **caractérisé en ce que** ledit au moins un monosaccharide et ledit au moins un oligosaccharide ont une pureté supérieure à 96-98%.

6. Mélange de principes actifs selon l'une des revendications précédentes, **caractérisé en ce que** ledit au moins un monosaccharide et ledit au moins un oligosaccharide sont obtenus par voie biotechnologique.

7. Composition à usage cosmétique **caractérisée en ce qu'**elle comprend un mélange de principes actifs selon l'une des revendications précédentes, lesdits au moins deux composants glucidiques dudit mélange de principes actifs étant contenus dans ladite composition selon un pourcentage en poids d'au moins 0,01%.

8. Composition à usage cosmétique selon la revendication 7, **caractérisée en ce que** lesdits au moins deux composants glucidiques sont contenus dans ladite composition selon un pourcentage en poids compris entre 0,01% et 10%.
